# EUROPEAN PATENT SPECIFICATION

(11) **EP 3 592 333 B1**
(45) Date of publication and mention of the grant of the patent: **03.04.2024**
(21) Application number: 18763649.3
(22) Date of filing: 20.02.2018
(51) Int. Cl.: A61K 9/16, A61K 47/32, A61K 47/26, A61K 31/216

(54) **A PHARMACEUTICAL COMPOSITION COMPRISING RACECADOTRIL AND PROCESS FOR PREPARING THE SAME**
PHARMAZEUTISCHE ZUSAMMENSETZUNG MIT RACECADOTRIL UND VERFAHREN ZUR HERSTELLUNG DAVON
COMPOSITION PHARMACEUTIQUE COMPRENANT DU RACÉCADOTRIL ET SON PROCÉDÉ DE PRÉPARATION

(30) Priority: 06.03.2017 IN 201721007797
(43) Date of publication of application: 15.01.2020
(73) Proprietor: Athena Pharmaceutiques SAS, 78430 Louveciennes (FR); Substipharm, 75016 Paris (FR)
(72) Inventor: CHAUDHARI, Mahendra B., Thane (West) 400 602 Maharashtra (IN); CHAUDHARI, Amol Y., Jalgaon 425 503 Maharashtra (IN); NEHETE, Nitin P., Dombivili (East) 421 201 Maharashtra (IN)
(74) Representative: Dr. Solf & Zapf Patent- und Rechtsanwalts PartG mbB
(86) International application number: PCT/IN2018/050085
(87) International publication number: WO 2018/163195

(56) References cited:
- EP-A1- 2 949 318
- WO-A1-01/97803
- WO-A1-2016/069871
- CN-A- 104 224 724
- US-A1- 2010 034 945
- US-B2- 6 919 093
- PETER MICHAEL GEARY: "The Co-Crystallisation of Sugars by the Supersaturation Process", Thesis submitted for the Degree of Doctor of Philosophy, September 2008 (2008-09), page 45, XP055556855,

## Description

### FIELD OF THE INVENTION

The present invention relates to a pharmaceutical composition comprising Racecadotril and co-crystallized sugar and a process or preparing the same.

### BACKGROUND OF THE INVENTION

Racecadotril (disclosed in EP038758B1) is an antidiarrheal drug which acts as an enkephalinase inhibitor which acts by reducing the secretion of water and electrolytes into the intestine. Chemically it is (RS)-Benzyl N-[3-(acetylthio)-2-benzylpropanoyl]glycinate with following chemical structure.

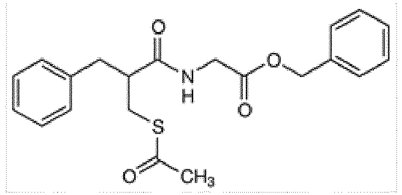

It is available commercially as Hidrasec^{®} and Tiorfan^{®} in dispersible tablet and sachet dosage forms. Racecadotril being a hydrophobic substance represents difficulties in preparing a suspension dosage form. Hence, it is formulated as a sachet and dispersible tablet dosage form.

EP1294372 discloses a dry powder composition comprising coated granules comprising Racecadotril in association with a pharmaceutically acceptable carrier.

EP2749270 discloses a dispersible tablet comprising Racecadotril whose taste is masked wherein the taste is masked by coating with an acrylic acid polymer or a cellulose polymer by wet granulation method without being mixed with any excipient.

In the existing dosage forms, the drug Racecadotril is coated, although there is no requirement for the same in terms of good palatability, sensitivity to hydrolysis or other environmental conditions, and product appearance. Specifically, racecadotril, when mixed with sweetening agents does not need any coating to show a good palatability. Further, the drug is not sensitive to hydrolysis or any other in vivo degradation process or environmental conditions like moisture and temperature. Also, there is no necessity of any coating to improve the product appearance as well.

Moreover, coated formulations as disclosed in the available dosage forms and disclosures come with additional process requirements and limitations. Generally, the coating process is costly and involves an extra step in manufacturing a simple dosage form like sachet. Also, the selection of coating process and the excipient for the same is difficult because of the hydrophobicity of Racecadotril.

Also, generally, the coatings must be stable and strong enough to survive the handling of the formulation, and must follow the fine contours of embossed characters on the formulation. The coated dosage form results into various defects like Picking and sticking (when coating removes a piece of the formulation from the core), Bridging (coating fills in the lettering or logo on the formulation), Erosion, Twinning (wherein two or more units get adhered to each other because of the coating), Peeling and Frosting (the coating peels away), Blistering (rapid evaporation of solvent from the coated formulation) or Orange peel (coating texture that resembles the surface of an orange). The manufacturing hurdles of the coating process and its effects during the shelf-life of the formulation suggest avoiding the coating process unless absolutely necessary.

CN 104224724 A discloses Racecadotril granules and a preparation method thereof. The granules are prepared from the following raw materials by weight: 0,1-2 parts of Racecadotril, 93-99 parts of an excipient, 0,02-2 parts of a correcting agent, 0,01-0,1 parts of an adhesive and 0,05-1 parts of a suspending agent. The correcting agent, for example stevia, sodium saccharin, aspartame, is used to mask the taste of the Racecadotril.

Accordingly, there is a need for easy process to manufacture a sachet dosage form for Racecadotril. Also, the process should be easy to develop and should not involve an unessential and complex coating process.

### SUMMARY OF THE INVENTION

To achieve the foregoing and other objects and needs, the present invention provides a pharmaceutical composition comprising Racecadotril and co-crystallized sugar and a process for preparing the same.

According to the invention, the pharmaceutical composition comprises Racecadotril, co-crystallized sugar and pharmaceutically acceptable inert excipients, wherein the ratio of Racecadotril to co-crystallized sugar is from about 1: 10 to about 1: 150, and wherein the co-crystallized sugar is co-crystallized sucrose.

In a preferred embodiment, the present invention provides a pharmaceutical composition comprising:
about 0.5 % to about 2 % w/w of Racecadotril;
about 40 % to about 99 % w/w of co-crystallized sugar;
about 0.1 % to about 5 % w/w of disintegrant;
about 0.05 % to about 2 % w/w of glidant,
wherein the co-crystallized sugar is co-crystallized sucrose.

According to the invention, the process for preparing a pharmaceutical composition comprising Racecadotril comprises the steps of:
(a) Sifting Racecadotril, a portion of co-crystallized sugar and pharmaceutically acceptable inert excipients to form a mixture;
(b) Adding a solvent in polyvinyl pyrrolidone to form a solution or suspension;
(c) Granulating the mixture formed in step (a) with solution or suspension formed in step (b) to form granules;
(d) Drying the granules formed in step (c);
(e) Blending another portion of co-crystallizedsugar with silicon dioxide to form intermediate blend;
(f) Mixing the dried granules formed in step (d) and the intermediate blend formed in step (e) to form a final blend;
(g) Filling the final blend formed in step (f) in a suitable sized sachet;
wherein the ratio of Racecadotril to co-crystallized sugar is from about 1: 10 to about 1: 150, and wherein the co-crystallized sugar is co-crystallized sucrose.

### BRIEF DESCRIPTION OF THE DRAWINGS

The advantages and features of the present invention will become better understood with reference to the following detailed description and claims taken in conjunction with the accompanying drawing, in which:

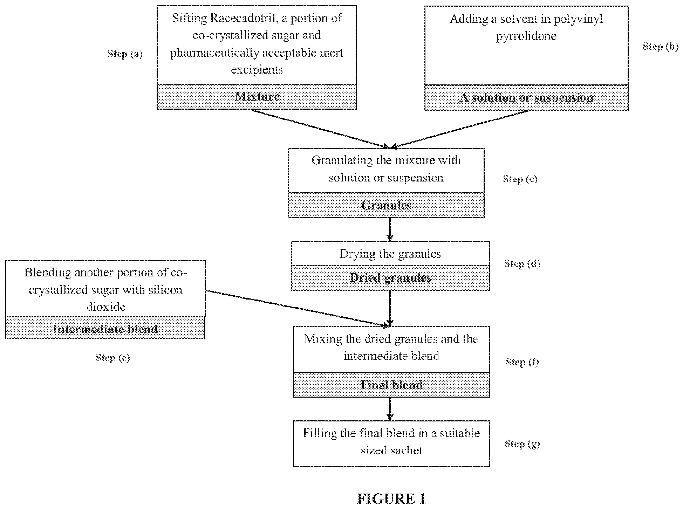

FIG. 1 illustrates a process flow for preparation of a pharmaceutical composition comprising Racecadotril, in accordance with an exemplary embodiment of the present invention.

### DETAILED DESCRIPTION OF THE INVENTION

The exemplary embodiments described herein in detail for illustrative purposes are subject to many variations in structure and design. It should be emphasized, however, that the present invention is not limited to a pharmaceutical composition comprising Racecadotril and preparation process for the same, as shown and described. Also, it is to be understood that the phraseology and terminology used herein is for the purpose of description and should not be regarded as limiting.

The use of terms "including," "comprising," or "having" and variations thereof herein is meant to encompass the items listed thereafter and equivalents thereof as well as additional items. Further, the terms, "a" and "an" herein do not denote a limitation of quantity, but rather denote the presence of at least one of the referenced item.

The present invention provides a pharmaceutical composition (hereinafter referred to as the "composition") comprising Racecadotril, co-crystallized sugar and pharmaceutically acceptable inert excipients, wherein the ratio of Racecadotril to co-crystallized sugar is from about 1 : 10 to about 1 : 150, and wherein the co-crystallized sugar is co-crystallized sucrose. The composition of the present invention is easy to prepare and does not involve any complex steps like coating. The invention specifically relates to a pharmaceutical composition in a sachet dosage form.

In an aspect of the invention, the pharmaceutical composition is in the form of solid oral dosage forms. The solid dosage form may be tablet, capsule, pills, sachets, lozenges, granules, powder and the like. Preferably, the pharmaceutical composition is in the form of a sachet.

As used herein, the term Racecadotril includes free base as well as its pharmaceutically acceptable salts, enantiomers, polymorphic forms.

Co-crystallized sugar is prepared by co-crystallization technique. This technique involves spontaneous crystallization of a supersaturated sugar solution and a second ingredient by agitating it while cooling. This process produces an agglomerated sponge-like structure, with vastly increased surface area. The second ingredient is an integral part of the structure's matrix.

According to the invention, co-crystallized sugar is co-crystallized sucrose. The commercially available grades of co-crystallized sugars may be used for the purpose of carrying out the invention.

The composition of the present invention comprises the ratio of Racecadotril to co-crystallized sugar from about 1: 10 to about 1: 150. Preferably, the ratio of Racecadotril to co-crystallized sugar is 1: 10 or 1:20 or 1: 25 or 1: 50 or 1: 75 or 1: 85 or 1: 87,5 or 1: 90 or 1: 91 or 1:92 or 1: 93 or 1:94 or 1: 95 or 1: 96 or 1: 97 or 1: 98 or 1: 99 or 1: 100 or 1: 105 or 1: 110 or 1: 120 or 1: 125 or 1: 150 and the like.

By the term "pharmaceutically acceptable inert excipients", it is meant any of the components of a pharmaceutical composition other than active ingredients and which are approved by regulatory authorities or are generally regarded as safe for human or animal use.

Examples of pharmaceutically acceptable inert excipients include, but are not limited to diluents, binders, sweetening agent, disintegrants, solvents, lubricant, glidants and flavorants. A combination of excipients may also be used. The amount of excipient(s) employed will depend upon how much active agent is to be used. One excipient can perform more than one function as well.

Diluents include, but are not limited to confectioner's sugar, compressible sugar, dextrates, dextrin, dextrose, fructose, lactitol, mannitol, sucrose, starch, lactose, xylitol, sorbitol, talc, microcrystalline cellulose, calcium carbonate, calcium phosphate dibasic or tribasic, calcium sulphate and other materials known to one ordinarily skilled in the art and mixtures thereof. Co-crystallized sugars used in the present invention are used as diluents,
Binders include, but are not limited to, starches such as potato starch, wheat starch, corn starch (maize starch); microcrystalline celluloses; celluloses such as hydroxypropyl cellulose, hydroxyethyl cellulose, hydroxypropyl methylcellulose (HPMC), ethyl cellulose, sodium carboxymethylcellulose; natural gums like acacia, alginic acid, guar gum; liquid glucose, dextrin, povidone, syrup, polyethylene oxide, polyvinylpyrrolidone, poly-N-vinyl amide, polyethylene glycol, gelatin, poly propylene glycol, tragacanth and other materials known to one ordinarily skilled in the art and mixtures thereof.

The sweetening agents (also known as sweeteners) include, but are not limited to, aspartame, saccharin sodium, dipotassium glycyrrhizinate, stevia, thaumatin, acesulfame K, sucralose, and other materials known to one ordinarily skilled in the art and mixtures thereof.

The disintegrants include, but are not limited to, starch, croscarmellose sodium, polyvinyl pyrrolidone, sodium starch glycolate and other materials known to one ordinarily skilled in the art and mixtures thereof. Preferably, the disintegrant is polyvinyl pyrrolidone.

Solvents include, but are not limited to purified water, acetone, ethyl alcohol, isopropyl alcohol dichloromethane and other materials known to one ordinarily skilled in the art and mixtures thereof.

Lubricants may be selected from, but are not limited to, those conventionally known in the art such as Mg, Al or Ca or Zn stearate, polyethylene glycol, glyceryl behenate, mineral oil, sodium stearyl fumarate, stearic acid, hydrogenated vegetable oil, talc and other materials known to one ordinarily skilled in the art and mixtures thereof.

Glidants include, but are not limited to, silicon dioxide; magnesium trisilicate, powdered cellulose, starch, talc and tribasic calcium phosphate, calcium silicate, magnesium silicate, colloidal silicon dioxide, silicon hydrogel and other materials known to one ordinarily skilled in the art and mixtures thereof. Preferably, the glidant is colloidal silicon dioxide.

The flavorants include natural and artificial flavors. These flavors include, but are not limited to synthetic flavor oils and flavoring aromatics, and/or oils, oleo resins and extracts derived from plants, leaves, flowers, fruits, etc., and other materials known to one ordinarily skilled in the art and mixtures thereof. Representative flavor oils include: spearmint oil, cinnamon oil, peppermint oil, clove oil, bay oil, thyme oil, cedar leaf oil, oil of nutmeg, oil of sage, and oil of bitter almonds. Also useful are artificial, natural or synthetic fruit flavors such as vanilla, chocolate, coffee, cocoa and citrus oil, including lemon, orange, grape, lime and grapefruit and fruit essences including apple, pear, peach, strawberry, raspberry, cherry, plum, pineapple, apricot and so forth. These flavorings can be used individually or in admixture. Commonly used flavors also include mints such as peppermint, artificial vanilla, cinnamon derivatives, and various fruit flavors, whether employed individually or in admixture. Flavorings such as aldehydes and esters including cinnamyl acetate, cinnamaldehyde, citral, diethylacetal, dihydrocarvyl acetate, eugenyl formate, p-methylanisole, and so forth may also be used.

The present invention further relates to a pharmaceutical composition comprising:
about 0.5 % to about 2 % w/w of Racecadotril;
about 40 % to about 99 % w/w of co-crystallized sugar;
about 0.1 % to about 5 % w/w of disintegrant;
about 0.05 % to about 2 % w/w of glidant,
wherein the co-crystallized sugar is co-crystallized sucrose.

The present invention further relates to a process for preparing a pharmaceutical composition comprising Racecadotril. Conventional processes for preparing the compositions like wet granulation, dry granulation and direct compression can be used.

According to the present invention, the process for preparing a pharmaceutical composition comprising Racecadotril comprises the steps of:
(a) Sifting Racecadotril, a portion of co-crystallized sugar and pharmaceutically acceptable inert excipients to form a mixture;
(b) Adding a solvent in polyvinyl pyrrolidone to form a solution or suspension;
(c) Granulating the mixture formed in step (a) with solution or suspension formed in step (b) to form granules;
(d) Drying the granules formed in step (c);
(e) Blending another portion of co-crystallized sugar with silicon dioxide to form intermediate blend;
(f) Mixing the dried granules formed in step (d) and the intermediate blend formed in step (e) to form a final blend;
(g) Filling the final blend formed in step (f) in a suitable sized sachet,
wherein the ratio of Racecadotril to co-crystallized sugar is from about 1 : 10 to about 1 : 150, and wherein the co-crystallized sugar is co-crystallized sucrose.

Mixing of the excipients can be performed in a conventional device used for mixing of powders, such as for example motionless (passive) mixers, fluidized bed, diffusion, bi-conicdiffusion, uniconic, biconic, turbular, cubic, planetary, Y-, V-shaped, and low shear or high shear mixers.

The granulation operation may be performed using apparatus such as, for example, a fluidized-bed granulator, an agitation granulator, a biaxial granulator, or the like.

Generally, the granulated mixture is dried after granulation in step (c) in any pharmaceutical acceptable manner. Drying of the granulate for example can be performed in one of the following ways: trays, fluid bed, and microwave assist/vacuum/gas stripping (one pot processing).

The granulation may be carried out by suitable aqueous or non-aqueous solvents.

The pharmaceutical composition according to the invention provides a further advantage of not having to coat the drug Racecadotril. As described above, there is no requirement for the coating Racecadotril for achieving good palatability, sensitivity to hydrolysis or other environmental conditions, and product appearance. Accordingly, the process of the invention avoids the process of coating, thereby avoiding additional coating process.

The description of the present invention of pharmaceutical composition comprising Racecadotril is further illustrated by the following non-limiting example. However, a person skilled in the art would recognize that the specific example is intended to illustrate, not limit, the scope of the present invention.

### EXAMPLES:

### EXAMPLE 1: Pharmaceutical composition according to the present invention

**Table 1: Pharmaceutical composition comprising Racecadotril**

| **Ingredients** | **% W/W** | **10 mg /sachet** | **30 mg /sachet** |
|---|---|---|---|
| Racecadotril | 1.00 | 10.00 | 30.00 |
| Co-crystallized sucrose | 41.50 | 415.00 | 1245.00 |
| Polyvinyl pyrrolidone | 0.15 | 0.50 | 15.00 |
| Purified water | Q.S | Q.S | Q.S |
| Co-crystallized sucrose | 57.25 | 572.50 | 1717.50 |
| Colloidal silicon dioxide | 0.10 | 1.00 | 3.00 |
| Total | 100 | 1000 | 3000 |

### Manufacturing process:

### 1. Preparation of drug granules:

Co-crystallized sucrose was milled in a suitable mill. It was sifted with intermediate addition of Racecadotril & colloidal silicon dioxide in a suitable sifter. The sifted mass was added in a mixer and mixed.

Purified water was added in polyvinyl pyrrolidone to form a suspension. The binder suspension was added to the dry mix in a suitable granulator. The wet mass was dried in a suitable dryer. The dried granules were sifted and milled. The dried granules were transferred to a suitable blender and mixed thoroughly.

### 2. Preparation of bulk granules:

Another portion of co-crystallized sucrose was milled in a suitable mill. Racecadotril and milled co-crystallized sucrose were sifted. The sifted mass was blended properly. Blended mass of drug granules materials was transferred in to the blender containing intermediate blend and mixed properly. The blended material in the above step was sifted again and mixed properly.

### 3. Sachets filling:

Racecadotril bulk granules were filled in a suitable sachet.

The packing details were as follows:

**Table 2: Packing details for pharmaceutical composition comprising Racecadotril**

| SN | PARAMETERS | STANDARD | LIMITS |
|---|---|---|---|
| **Racecadotril sachets 10 mg** | | | |
| 1 | Appearance | White to off-white granules aluminum sachets filled in heat sealed | |
| 2 | Average fill weight | 1000.00 mg | 1000 mg ± 5 % (950 - 1050 mg) |

| **Racecadotril sachets 30 mg** | | | |
|---|---|---|---|
| 1 | Appearance | White to off-white granules aluminum sachets filled in heat sealed | |
| 2 | Individual Sachet weight | 3000 mg | 3000 mg ± 5 % (2850 - 3150 mg) |

## Claims

1. A pharmaceutical composition comprising Racecadotril, co-crystallized sugar and pharmaceutically acceptable inert excipients, wherein the ratio of Racecadotril to co-crystallized sugar is from about 1 : 10 to about 1 : 150, and wherein the co-crystallized sugar is co-crystallized sucrose.

2. The pharmaceutical composition according to claim 1, wherein the composition is uncoated.

3. The pharmaceutical composition according to claim 1, wherein the pharmaceutically acceptable inert excipients comprise diluents, binders, sweetening agent, disintegrants, solvents, lubricant, glidants and flavorants.

4. A pharmaceutical composition comprising:
about 0.5 % to about 2 % w/w of Racecadotril;
about 40 % to about 99 % w/w of co-crystallized sugar;
about 0.1 % to about 5 % w/w of disintegrant;
about 0.05 % to about 2 % w/w of glidant,
wherein the co-crystallized sugar is co-crystallized sucrose.

5. The pharmaceutical composition according to claim 4, wherein the composition is a solid dosage form.

6. The pharmaceutical composition according to claim 4, wherein the composition is in the form of a sachet.

7. The pharmaceutical composition according to claim 4, wherein the disintegrant is polyvinyl pyrrolidone.

8. The pharmaceutical composition according to claim 4, wherein the glidant is colloidal silicon dioxide.

9. A process for preparing a pharmaceutical composition comprising Racecadotril, the process comprising the steps of:
(a) Sifting Racecadotril, a portion of co-crystallized sugar and pharmaceutically acceptable inert excipients to form a mixture;
(b) Adding a solvent in polyvinyl pyrrolidone to form a solution or suspension;
(c) Granulating the mixture formed in step (a) with solution or suspension formed in step (b) to form granules;
(d) Drying the granules formed in step (c);
(e) Blending another portion of co-crystallized sugar with silicon dioxide to form intermediate blend;
(f) Mixing the dried granules formed in step (d) and the intermediate blend formed in step (e) to form a final blend;
(g) Filling the final blend formed in step (f) in a suitable sized sachet;
wherein the ratio of Racecadotril to co-crystallized sugar is from about 1 : 10 to about 1 : 150, and wherein the co-crystallized sugar is co-crystallized sucrose.

10. The process according to claim 9, wherein the granulation process is aqueous granulation.

11. The process according to claim 9, wherein the granulation process is non-aqueous granulation.

## Patentansprüche

1. Pharmazeutische Zusammensetzung, umfassend Racecadotril, co-kristallisierten Zucker und pharmazeutisch zulässige inerte Hilfsstoffe, wobei das Verhältnis von Racecadotril zu co-kristallisiertem Zucker etwa 1:10 bis etwa 1:150 beträgt und wobei der co-kristallisierte Zucker co-kristallisierte Saccharose ist.

2. Pharmazeutische Zusammensetzung nach Anspruch 1, wobei die Zusammensetzung unbeschichtet ist.

3. Pharmazeutische Zusammensetzung nach Anspruch 1, wobei die pharmazeutisch zulässigen inerten Hilfsstoffe Verdünnungsmittel, Bindemittel, Süßungsmittel, Sprengmittel, Lösungsmittel, Gleitmittel, Gleitmittel und Geschmacksstoffe umfassen.

4. Pharmazeutische Zusammensetzung, umfassend:
etwa 0,5 Gew.-% bis etwa 2 Gew.-% Racecadotril;
etwa 40 Gew.-% bis etwa 99 Gew.-% an co-kristallisiertem Zucker;
etwa 0,1 Gew.-% bis etwa 5 Gew.-% Sprengmittel;
etwa 0,05 Gew.-% bis etwa 2 Gew.-% Gleitmittel,
wobei der co-kristallisierte Zucker co-kristallisierte Saccharose ist.

5. Pharmazeutische Zusammensetzung nach Anspruch 4, wobei die Zusammensetzung eine feste Darreichungsform ist.

6. Pharmazeutische Zusammensetzung nach Anspruch 4, wobei die Zusammensetzung in Form eines Beutels vorliegt.

7. Pharmazeutische Zusammensetzung nach Anspruch 4, wobei das Sprengmittel Polyvinylpyrrolidon ist.

8. Pharmazeutische Zusammensetzung nach Anspruch 4, wobei das Gleitmittel kolloidales Siliciumdioxid ist.

9. Verfahren zur Herstellung einer pharmazeutischen Zusammensetzung, die Racecadotril enthält, wobei das Verfahren die folgenden Schritte umfasst:
(a) Sieben von Racecadotril, einem Teil des co-kristallisierten Zuckers und pharmazeutisch zulässigen inerten Hilfsstoffen, um eine Mischung zu bilden;
(b) Zugabe eines Lösungsmittels in Polyvinylpyrrolidon zur Bildung einer Lösung oder Suspension;
(c) Granulieren der in Schritt (a) gebildeten Mischung (a) mit der in Schritt (b) gebildeten Lösung oder Suspension zur Bildung von Granalien;
(d) Trocknen der in Schritt (c) gebildeten Granalien (c);
(e) Mischen eines weiteren Teils des co-kristallisierten Zuckers mit Siliziumdioxid zur Bildung einer Zwischenmischung;
(f) Mischen der in Schritt (d) gebildeten getrockneten Granalien und der in Schritt (e) gebildeten Zwischenmischung um eine finale Mischung zu bilden;
(g) Abfüllen der finalen, in Schritt (f) gebildeten Mischung in einen Beutel von geeigneter Größe;
wobei das Verhältnis von Racecadotril zu co-kristallisiertem Zucker etwa 1:10 bis etwa 1:150 beträgt und wobei der co-kristallisierte Zucker co-kristallisierte Saccharose ist.

10. Das Verfahren nach Anspruch 9, wobei das Granulationsverfahren eine wässrige Granulation ist.

11. Das Verfahren nach Anspruch 9, wobei das Granulationsverfahren eine nichtwässrige Granulation ist.

## Revendications

1. Composition pharmaceutique comprenant du racécadotril, du sucre co-cristallisé et des excipients inertes pharmaceutiquement acceptables, dans laquelle le rapport entre le racécadotril et le sucre co-cristallisé est d'environ 1 : 10 à environ 1 : 150, et dans laquelle le sucre co-cristallisé est du saccharose co-cristallisé.

2. Composition pharmaceutique selon la revendication 1, dans laquelle la composition n'est pas revêtue.

3. Composition pharmaceutique selon la revendication 1, dans laquelle les excipients inertes pharmaceutiquement acceptables comprennent des diluants, des liants, des édulcorants, des désintégrants, des solvants, des lubrifiants, des agents de glisse et des aromatisants.

4. Composition pharmaceutique comprenant
environ 0,5 % à environ 2 % p/p de racécadotril ;
d'environ 40 % à environ 99 % p/p de sucre co-cristallisé ;
d'environ 0,1 % à environ 5 % p/p de désintégrant ;
d'environ 0,05 % à environ 2 % p/p d'agent de glisse,
dans lequel le sucre co-cristallisé est du saccharose co-cristallisé.

5. Composition pharmaceutique selon la revendication 4, dans laquelle la composition est une forme de dosage solide.

6. Composition pharmaceutique selon la revendication 4, dans laquelle la composition se présente sous la forme d'un sachet.

7. Composition pharmaceutique selon la revendication 4, dans laquelle le désintégrant est la polyvinylpyrrolidone.

8. Composition pharmaceutique selon la revendication 4, dans laquelle l'agent de glisse est du dioxyde de silicium colloïdal.

9. Procédé de préparation d'une composition pharmaceutique comprenant du racécadotril, le procédé comprenant les étapes suivantes:
(a) Tamiser le racécadotril, une partie du sucre co-cristallisé et des excipients inertes pharmaceutiquement acceptables pour former un mélange;
(b) Ajouter un solvant dans la polyvinylpyrrolidone pour former une solution ou une suspension;
(c) Granuler le mélange formé à l'étape (a) avec la solution ou la suspension formée à l'étape (b) pour former des granulés;
(d) Sécher les granulés formés à l'étape (c);
(e) Mélanger une autre partie du sucre co-cristallisé avec du dioxyde de silicium pour former un mélange intermédiaire;
(f) Mélanger les granulés séchés formés à l'étape (d) et le mélange intermédiaire formé à l'étape (e) pour former un mélange final;
(g) Remplir le mélange final formé à l'étape (f) dans un sachet de taille appropriée;
dans lequel le rapport racécadotril/sucre co-cristallisé est d'environ 1 : 10 à environ 1 : 150, et dans lequel le sucre co-cristallisé est du saccharose co-cristallisé.

10. Procédé selon la revendication 9, dans lequel le processus de granulation est une granulation aqueuse.

11. Procédé selon la revendication 9, dans lequel le processus de granulation est une granulation non aqueuse.
